# EUROPEAN PATENT APPLICATION

(11) **EP 2 154 240 A1**
(43) Date of publication of application: **17.02.2010**
(21) Application number: 08161947.0
(22) Date of filing: 06.08.2008
(51) Int. Cl.: C12N 5/00

(54) **PRF in cell and tissue culture**

(71) Applicant: UToC B.V. i.o, 1077 VG Amsterdam (NL)
(72) Inventor: The designation of the inventor has not yet been filed
(74) Representative: Hatzmann, Martin

(57) **Abstract**

The invention relates to a method to increase growth or differentiation in vitro in a cell or tissue culture comprising culturing said cell(s) or tissue and applying pulsed radiofrequency (PRF) to said cell or tissue. In said method the cells or tissue are derived from a plant or an animal (including human) and preferably the cells are undifferentiated cells. Also part of the invention is a method as described herein wherein the cells or tissue are derived from a plant and in which differentiation implies the outgrowth of roots and/or shoots or of somatic embryos. The invention further comprises the use of PRF for increasing the growth or differentiation in *in vitro* cell or tissue culture.

The invention also comprises a cell or tissue culture treated according to a method according to the invention. Preferably such a cell or tissue culture is derived from a plant. Then, in yet another embodiment, the invention is directed to a plant grown from such a cell or tissue culture. Alternatively, the invention comprises a cell culture that is treated according a method of the invention wherein the cells are derived from an animal, preferably a human.

## Description

### FIELD OF THE INVENTION

The invention relates to the field of biotechnology, more particularly to the field of cell and tissue culture, more specifically to methods to improve cell and tissue culture conditions and differentiation of cells.

### BACKGROUND TO THE INVENTION

In the past thirty years the field of cell and tissue culture has become increasingly important in biotechnology.

Plant tissue culture involves the growing of plant tissue from plant material taken from a source plant. It has been found that plants can reproduce whole plants from fragments of plant material when given a nutrient media capable of supporting growth and appropriate hormone control. For plants, in vitro techniques were developed initially to demonstrate the totipotency of plant cells predicted by Haberlandt in 1902. Totipotency is the ability of a plant cell to perform all the functions of development, which are characteristic of a zygote, i.e., ability to develop into a complete plant. In 1902, Haberlandt reported culture of isolated single palisade cells from leaves in Knop's salt solution enriched with sucrose. The cells remained alive for up to 1 month, increased in size, accumulated starch but failed to divide. Efforts to demonstrate totipotency led to the development of techniques for cultivation of plant cells under defined conditions.

This was made possible by the brilliant contributions from RJ. Gautheret in France and P.R. White in the U.S.A. during the third and the fourth decades of 20^{th} century. Most of the modern tissue culture media derive from the work of Skoog and coworkers during 1950s and 1960s.

The first embryo culture, although crude, was done by Hanning in 1904; he cultured nearly mature embryos of certain crucifers and grew them to maturity. The technique was utilised by Laibach in 1925 to recover hybrid progeny from an interspecific cross in Linum. Subsequently, contributions from several workers led to the refinement of this technigue.

Haploid plants from pollen grains were first produced by Maheshwari and Guha in 1964 by culturing anthers of *Datura.* This marked the beginning of anther culture or pollen culture for the production of haploid plants. The technique was further developed by many workers, more notably by JP. Nitch, C. Nitch and coworkers. These workers showed that isolated microspores of tobacco produce complete plants.

Plant protoplasts are naked cells from which cell wall has been removed. In 1960, Cocking produced large quantities of protoplasts by using cell wall degrading enzymes. The techniques of protoplast production have now been considerably refined. It is now possible to regenerate whole plants from protoplasts and also to fuse protoplasts of different plant species. In 1972, Carlson and coworkers produced the first somatic hybrid plant by fusing the protoplasts of *Nicotiana glauca* and *N. langsdorfii.* Since then many divergent somatic hybrids have been produced.

A successful establishment of callus cultures depended on the discovery during mid-thirties of IAA (idole-3-acetic acid), the endogenous auxin, and of the role of B vitamins in plant growth and in root cultures. The first continuously growing callus cultures were established from cambium tissue in 1939 independently by Gautheret, White and Nobecourt. The subsequent discovery of kinetin by Miller and coworkers in 1955 enabled the initiation of callus cultures from differentiated tissues. Shoot bud differentiation from tobacco pith tissues cultured in vitro was reported by Skoog in 1944, and in 1957 Skoog and Miller proposed that root-shoot differentiation in this system was regulated by auxin-cytokinin ratio.

The first plant from a mature plant cell was regenerated by Braun in 1959. Development of somatic embryos was first reported in 1958- 1959 from carrot tissues independently by Reinert and Steward.

Thus within a brief period, the tissue culture techniques have made a great progress. From the sole objective of demonstrating the totipotency of differentiated plant -cells, the technique now finds application in both basic and applied researches in a number of fields of enquiry.

The beginning of animal (including human) tissue culture can be traced to 1880 when Arnold showed that leucocytes can divide outside the body. Later, in 1903, Jolly studied the behaviour of animal tissue explants immersed in serum, lymph or ascites fluid. In 1907, Harrison cultured frog tadpole spinal chord in a lymph drop hanging from a cover slip into the cavity on a microscopic slide; this is regarded as the turning point. A few years later, in 1913, Carrel developed a complicated methodology for maintaining cultures free from contamination, especially by bacteria.

In 1952 Gey got the first continuous cell line going called HeLa, which was later cloned by Puck using X-rayed feeder layers. Parker's use of antibiotics in 1961 made it possible to keep these cell lines going. In the late 50s and early 60s development of defined media made it possible to have serum-free media.

Both in plant and animal tissue and cell culture, since the 1960s many advances have been obtained, not only in the origins of cells and tissues that can be kept in culture, but also in the applications of tissue and cell culture. Plant tissue culture is used widely in plant science; it also has a number of commercial applications. Applications include:
- Micropropagation is widely used in forestry and in floriculture. Micropropagation can also be used to conserve rare or endangered plant species.
- A plant breeder may use tissue culture to screen cells rather than plants for advantageous characters, e.g herbicide resistance/tolerance.
- To cross-pollinate distantly related species and then tissue culture the resulting embryo which would otherwise normally die (Embryo Rescue).
- For production of doubled monoploid plants from haploid cultures to achieve homozygous lines more rapidly in breeding programmes, usually by treatment with colchicine which causes doubling of the chromosome number.
- As a tissue for transformation, followed by either short-term testing of genetic constructs or regeneration of transgenic plants.
- Certain techniques such as meristem tip culture may be employed that can be used to produce clean plant material from virused stock, such as potatoes and many species of soft fruit and ornamental plants.

In animal cell culture, mass culture of animal cell lines is fundamental to the manufacture of viral vaccines and many products of biotechnology. Biological products produced by recombinant DNA (rDNA) technology in animal cell cultures include enzymes, hormones, immunobiologicals (monoclonal antibodies, interleukins, lymphokines), and anticancer agents. Powerful developments in the multidisciplinary field of tissue engineering have yielded a novel set of tissue replacement parts and implementation strategies. Scientific advances in biomaterials, stem cells, growth and differentiation factors, and biomimetic environments have created unique opportunities to fabricate tissues in the laboratory from combinations of engineered extracellular matrices ("scaffolds"), cells, and biologically active molecules.

In this rapidly evolving field there is still need for new technologies that improve the efficacy and speed of tissue and cell cultures.

### SUMMARY OF THE INVENTION

The invention now is directed to the use of Pulse Radio Frequency (PRF) for stimulating cell and/or tissue culture. Thus the invention comprises a method to increase growth or differentiation in vitro in a cell or tissue culture comprising culturing said cell(s) or tissue and applying PRF to said cell or tissue. In said method the cells or tissue are derived from a plant or an animal (including human) and preferably the cells are undifferentiated cells. Also part of the invention is a method as described herein wherein the cells or tissue are derived from a plant and in which differentiation implies the outgrowth of roots and/or shoots or of somatic embryos.

In another embodiment in such a method the parameters for PRF are as follows:
a. Frequency: 50,000 - 1,000,000 Hz, preferably 150,000 - 500,000 Hz
b. Pulse duration: 0.1-100 msec, preferably 5-20 msec.
c. Pulse frequency: 1- 20/sec, preferably 1 - 3/sec
d. Voltage: 1-100 V per cm distance between electrodes
e. Exposure time: 2-180 minutes

In another embodiment the invention comprises the use of PRF for increasing the growth or differentiation in in *vitro* cell or tissue culture.

In yet another embodiment the invention comprises a cell or tissue culture treated according to a method according to the invention. Preferably such a cell or tissue culture is derived from a plant. Then, in yet another embodiment, the invention is directed to a plant grown from such a cell or tissue culture.

Alternatively, the invention comprises a cell culture that is treated according a method of the invention wherein the cells are derived from an animal, preferably a human.

### LEGENDS TO THE FIGURES

Fig. 1. Generator and containers used for PRF treatment of plants.
Fig. 2. Explants of *Sorbus terminalis* treated with PRF with autoclavable electrodes.
Fig. 3. Sterile explants treated between electrodes (left panel) and tissue cultured *Cydonia oblonga* plants after PRF treatment (right panel).
Fig. 4. *Acer pseudoplatanus* explants treated with PRF: stems are positioned between the two electrodes.
Fig. 5. Evaluation of *Viola wittrockiana* tissue culture plants: after PRF treatment (1 and 3) they seem more homogeneous than the control (front row K). Different colours are being used for different plant sizes.
Fig. 6. Top panel: tissue culture *Delphinium* plants treated with PRF (22V or 45 V) showed more roots and were stronger and better in quality than the controls untreated plants (right). Bottom panel: differences in height of PRF treated (right) and untreated plants (left).
Fig. 7. Explants of *Sorbus terminalis* treated with PRF in autoclavable electrodes.
Fig. 8. Left panel: PRF treatment with needles. Right panel: Shoot and root regeneration 3 weeks after PRF treatment of plant cells. Treated cells are marked (+) and untreated cells (-) on the petridishes.
Fig. 9. Root formation in *Acer pseudoplatanus.* Left: Control untreated plants (only 3 out of 10 plants did survive) Right: PRF Treated plants (20V) with root formation (in 5 out of 10 plants).
Fig. 10. Adventitious root and shoot formation after PRF treatment (+) and the untreated controls (-).
Fig. 11. Somatic embryo formation and root formation on hormone free medium, after PRF treatment.
Fig. 12. Root development in Cydonia oblonga. Right: PRF treated plants, Left: untreated controls.
Fig. 13. Root formation of treated and untreated (Kontrole) *Delphinium* plants.

### DETAILED DESCRIPTION

PRF (pulsed radiofrequency) is used in medicine as a clinically proven method to alleviate pain in cases where pain sensation is due to or transported via peripheral nerves (such as in case of pain caused by pinching a nerve by a slipped disc of the spinal column, facial pain, trauma, etc.). PRF, just like RF, works through applying an electrical AC current to the vicinity of a nerve. Usually a frequency of 400.000 - 500.000 Hz is used, but the range may vary from 50.000 to 1.000.000 Hz. With PRF, current is delivered in pulses of short duration (1-100 msec) separated by a silent period of about 0,1 to 1 sec. In PRF, in contrast to continuous RF, the heat that is generated at the tip of the electrode during the active phase of the duty cycle is dissipated during the resting phase of zero, or of appreciably lower voltage. It is allowable that the temperature may briefly rise with up to 5° C during the active phase of the duty cycle (the so-called heat spikes), although the biological effects of these ultrashort and moderate rises in temperature are not known. Furthermore, the spread of heat during a heat spike has been predicted to be minimal (< 0.2 mm in human tissue), thereby practically outruling that a thermal effect occurs. As for yet, there is no conclusive theory explaining and supporting the observed clinical effects of PRF.

Continuous Radiofrequency (RF) electrical fields have been applied to cell cultures as described in Cleary, S.F. et al., 1996, FASEB J., 10:913-919, and to pathogenic micro-organisms on plants as described in WO 02/39786. Although in many cases it is indicated that the development of heat by application of the electrical field and thus an increase of the ambient temperature is an essential feature of the treatment, there are also indications that radiofrequency treatment may affect membrane signal transduction. Cahana A. et al. (J. Pain, 2003, 4:197-202) exposed hippocampal slice cultures to continuous RF and to PRF at 42 °C. They found that PRF had a transient effect and RF a lasting effect on evoked synaptic activity. In addition there was distance-dependent tissue destruction over a very short distance, and this was more pronounced in the continuous RF group. In conclusion they stressed the importance of the various parameters of PRF.

Much research to effects of pulsed radiofrequencies and/or electromagnetic radiofrequency fields produced by cell-phones has been performed in the last couple of years. However, although in some of these studies minor effects of the radiation have been reported, in general it has been concluded that cell-phone produced radiation has no significant effects on cells of the human body.

It is therefore surprisingly found by the inventors that PRF affects the cell growth and/or the differentiation of cells and tissues in *in vitro* cultures. Without being bound to theory, the hypothesis is that PRF treatment causes a flux or a change over the cell membrane, which in turn may have an effect on signalling molecules that are responsible for signal transduction (e.g. hormonal signal transduction in plant cells). Indeed has radiofrequency been used in electroporation techniques, where the treatment has been used for transiently permeabilizing cell membranes to enable introduction of nucleic acids in cells (see e.g. He, H. et al., 2006, Bioelectrochem. 68:89-97). However, in contrast to other electrical and radiofrequency stimulations, application of PRF does not or hardly cause any high temperature related effects on the treated cells.

Plant cells and tissue cultures in the present invention are deemed to include, amongst others, undifferentiated as well as differentiated cells: single cells, meristems, protoplasts, callus cells, cell suspensions, somatic embryo's, in vitro propagated explants, shoot and root cultures, anthers, microspores, egg cells, flower and tapetum cells.

Animal cells and cell culture comprise differentiated and undifferentiated cells, such as embryonal or umbilical stem cells or other totipotent or pluripotent cells; immortal cell lines, such as hybridomas; and cell cultures of HeLA, 3T3, Jurkat, HEK 293, CHO or other cells, such as listed on http://www.biotech.ist.unige.it/cldb/cname-1c.html, most of which are obtainable from commercial suppliers such as Sigma Aldrich® and Promega®.

It is shown herein that treatment of PRF enhances cell growth in plant tissue cultures, and also induces or enhances differentiation of cell in such a culture to form differentiated tissues such as somatic embryos, roots and/or shoots. It has also been observed that adventitious root formation is enhanced, thereby increasing the root system of the plantlets and enabling a better and faster growth of the plant. The secondary effect of these changes is that the plants developing from PRF-treated cells and/or tissue are more vigorous than control plants. This especially shows under stress conditions, such as pathogen infection and drought stress. A further secondary effect from PRF treatment is that it increases the homogeneity between plants that have been treated simultaneously. This shows in a more synchronized growth and following from this less spread in flowering time.

Regeneration of adventitious organs (roots or shoots) on starting material such as explants or callus tissue and regeneration of plants from suspensions of, or even single, cells or protoplasts used as starting material is a technique that is used frequently for seedless propagation. For the generation of transformed or transgenic plants, in vitro propagation is even considered a prerequisite, since it is the totipotency of individual plant cells that underlies most plant transformation systems.

To propagate plants from starting material in vitro, it is in principle necessary that at least one cell in the starting material is capable of regeneration. The ability to regenerate is for example determined by the genotype, the environmental conditions (nutrient supply, regulators and physical conditions) or the developmental stage of the plant, or combinations of these. It is well known that some families and genera have high regeneration ability: *Solanacea (Solanum, Nicotiana, Petunia, Datura,* and *Lycopersion), Crucifera (Lunaria, Brassica, Arabidopsis), Generiaceae (Achimenes, Saintpaulia, Streptocarpus) Compositae (Chicorium, Lactuca, Chrysantemum), Liliaceae (Lilium, Haworthia) Allium, Ornithogalum)* but others, such as many decorative plants, woody species such as shrubs, conifers or trees, especially fruit and nut trees, *Rosacea, Alstroemeria, Euphorbia,* and bulbs such as *Tulipa,* and others are notoriously difficult, even with in vitro techniques.

Regeneration in plants comprises the formation of new tissues containing both root and shoot meristems, separate shoot or root meristems, plant organs or organ primordia from individual cells or groups of cells. Regeneration in general mimics the process of normal cellular and organ differentiation that takes place during plant development and results in the formation of the different plant organs. In normal development, early in ontogony, cells and tissues of common lineage diverge into often contrasting paths of development as they respond to developmental signals. This ability to develop in response to a specific signal is also known as cellular competence or cellular potentiality. As competent cells become committed to particular paths of differentiation, they are not readily diverted into other pathways; this restriction of the developmental potentiality of cells is referred to as determination.

Plant cells or groups of cells that under normal conditions are unable to initiate the formation of certain plant organs, meristems or organ primordia can often be stimulated by extracellular stimuli modifying the differentiation stage of the cell. Extracellular diffusible factors have shown to be essential for cellular redifferentiation in plant cells (Siegel and Verbeke, 1989 Science 244, 580-582). The perception of these signals at the cellular surface and the intracellular signal transduction that finally result in changes in transcriptional regulation provides cells with the ability to respond to such extracellular stimuli. Regeneration can result in the formation of either a shoot alone or a root alone or both together. Only after redifferentiation of a cell or tissue, regeneration is possible that results in differentiated tissue that again comprises the necessary three-dimensional layout of the emerging plant, the apical-basal or shoot-root body plan from which the mature desired plant can develop.

Central in in vitro techniques for seedless propagation are phytohormones and other factors added to the culture medium that mimic these extracellular stimuli. For the process of regeneration of an original starting cell into a multicellular totipotent tissue underlying and preceding somatic embryogenesis or organogenesis *in vitro* in cell, tissue or explant cultures which lead to a fully differentiated plant again, in general a well balanced, and per plant species often different, phytohormone addition to the culture is required. Overall, a balance is required between auxins on the one hand and cytokinins on the other. After exogenous exposure to auxin (such as 2,4-dichlorophenoxyacetic acid (2,4-D), chloramben or dicamba) or cytokinin (such as 6-benzylaminopurine or zeatine) or both, cells or tissue react by development of the shoot-root body plan, for example by forming shoots and/or roots, sometimes readily, sometimes erratically especially when the proper balance between the hormones is not properly selected.

Regeneration *in* vitro and especially the manipulatable nature of in vitro culture thus depends mainly on the application of these two types of hormones, and also on the ability of the tissue to respond to phytohormonal changes during culture. In general, three phases of regeneration are recognisable. In the first phase, cells in the culture acquire "competence", which is defined as the ability (not capacity) to respond to hormonal signals of organ induction. The process of acquisition of said organogenic competence is often referred to as "dedifferentiation" of differentiated cells to acquire organogenic competence. The competent cells in the culture are canalised and determined for specific tissue and organ formation for re-entry of quiescent cells into cell cycle, and organisation of cell division along the lines of the shoot-root body plan to form specific primordia and meristems under the influence of the phytohormone balance through the second phase. Especially auxin is thought to be involved in specific regenerative signal transduction pathways for adventitious root initiation, whereas cytokinin is thought to be involved in specific regenerative signal transduction pathways for adventitious shoot initiation.

Then the morphogenesis, the growing of the plant to its fully differentiated state, proceeds independently of the exogenously supplied hormones during the third phase.

Although the general principles governing regeneration via addition of exogenous phytohormones are thus fairly well understood, designing working in vitro culture protocols finding the right balance, the right time of administration or the right type or subtype of said hormones for a great many individual species is still more or less a process of trial-and-error. However, as already indicated above, for in vitro regeneration or seedless propagation of a great many plant species is a large interest, especially for those that are in general hard to propagate.

The current invention now facilitates this process in the sense that - as is demonstrated in the experimental part - growth of the tissue culture and differentiation is enhanced by PRF treatment. In particular, the invention provides a culture method wherein the starting material can comprises an individual plant cell or protoplast or explant or plant tissue, materials which are commonly used in in vitro culture methods whereby the addition of phytohormones was thought to be axiomatic. Now such addition is no longer necessary or can be reduced, providing an easier way of in vitro culture, wherein not such an intricate balance between the additions of the various hormones has to be sought. Furthermore, as indicated above, the PRF treatment results in plants that are more vigorous. Also a larger homogeneity is observed, which is a large commercial advantage in the culture of ornamental and crop plants, because as a result the time of and to harvest can be determined better.

With regard to animal cell cultures a preliminary study was performed on human stem cells, which showed that gene expression of several genes that play a role in differentiation was increased. Thus, it is envisaged that PRF treatment will also stimulate growth and/or differentiation in animal cell cultures.

As the Examples show, the optimal parameters, one of which is the time of the PRF treatment, is different for the various cell or tissue cultures treated. Usual values are a frequency of about 400,000 Hz, a pulse duration of 10 msec and a pulse frequency of 2/secThere is however a wide variation in parameters that may be used:
Frequency: 50,000 - 1,000,000 Hz, preferably 150,000 - 500,000 Hz, more preferably 300,000 - 450,000 Hz
Pulse duration: 0.1- 100 msec, preferably 5-20 msec.
Pulse frequency: 1- 20/sec, preferably 1 - 3/sec
Voltage: 1-100 V per cm distance between electrodes
Exposure time: 2-180 minutes

Especially the applied voltage and the time of the treatment are parameters which should be adjusted to achieve the optimal treatment. Preferably, the voltage is between about 5 and about 50 V/cm, and more preferably between about 10 and about 40 V/cm. The treatment time is preferably about 5 to about 60 minutes, more preferably about 10 to about 30 minutes. As is shown in the Examples, we have found that the optimal treatment parameters (with respect to voltage and treatment time) differ between the cell or tissue cultures of various species and the medium in which these are maintained.

Further the duty cycle of the PRF treatment may be irregular, with varying pulse duration and pulse frequency, and the voltage may not be brought back to zero during the rest phase.

The RF Lesion Generators that are commercially available are only suitable for performing the present procedure if the impedance of the circuit is larger than 50 Ohms (such generators are e.g. obtainable from Neurotherm Inc, Middleton MA, USA; Valleylab, Boulder, Colorado, US or Traatek, Fort Lauerdale, Florida, USA). The Radionics 3C plus generator (Radionics, Burlington, MA), which is no longer commercially available, does not have this limitation.

The electrodes used can be plate electrodes of any size or needle electrodes. Different types are illustrated in the experimental section. Also the manner in which the electrodes are positioned in or near the cell or tissue culture can vary, also depending on the size of the culture and the housing and medium in which the cell or tissue culture is maintained. Examples are given in the experimental part, but a person skilled in the art will be able to determine which electrodes are best suited for a particular experimental setting and how to best position them.

### EXAMPLES

### Example 1 Description of the methods for tissue culture plants to induce rooting by PRF treatment

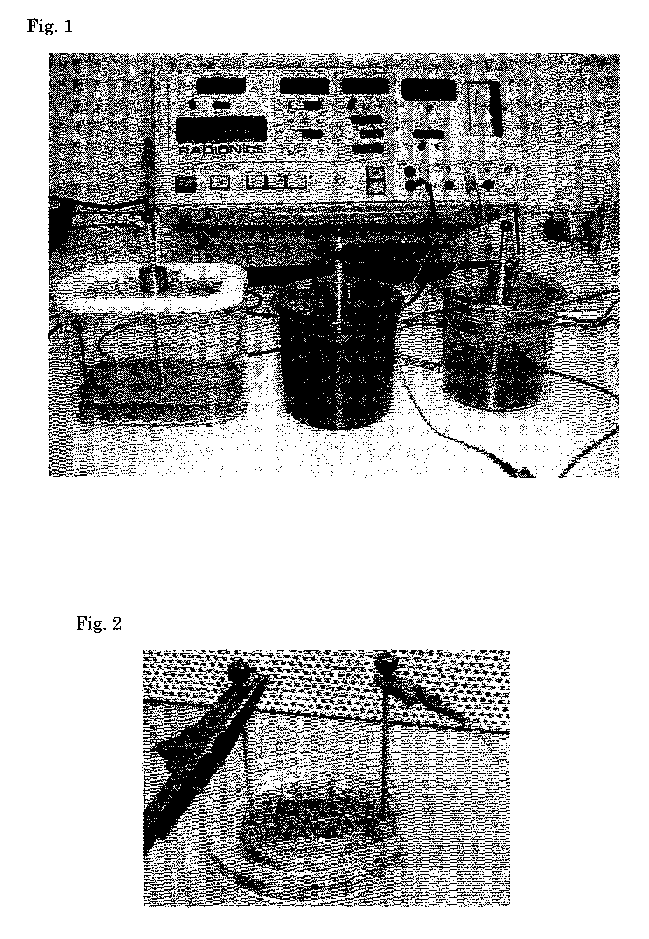

Tissue culture plants of flowering plants (e.g. *Campanula carpatia, Viola wittrockiana* and *Delphinium elatum* ) or fruit trees *(Cydonia oblonga*), and trees *(Acer pseudoplatanus, Sorbus torminalis*) were grown in sterile containers on normal tissue culture propagation medium.
In this culture medium the plants do normally not make any roots.
Before PRF treatment, the plants were taken out of the sterile container and the lower stem part was cut to make a fresh wounding site.
We have used a generator type RFG-3C plus (see Fig. 1), attached to a container, with following settings: pulses of 2 Hz, pulse radiofrequency 420.000 to 450.000 Hz, pulse duration 10 ms, voltage settings varying between 15-60 V/cm and varying duration of the PRF treatment (ranging from 10-30 minutes). Impedance of the used liquid tissue culture medium was found to be about 15-60 Ohm (depending on the chemical composition), and for solid agar medium up to 380 Ohm. For the woody species we have treated the plants longer (15-30 minutes) with higher Voltage settings (15- 40V/cm).

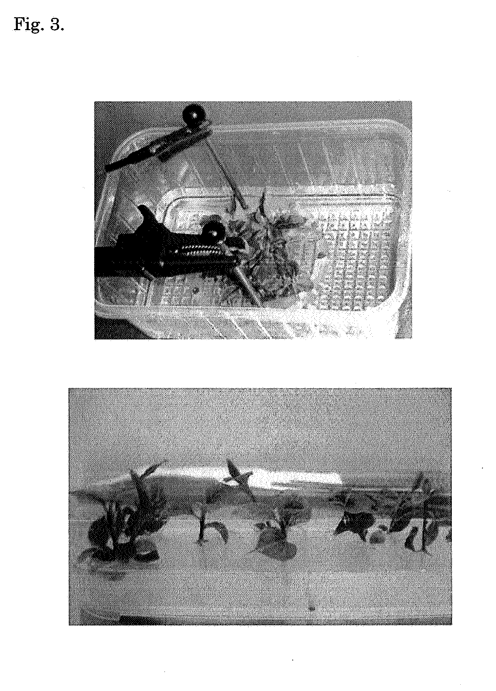

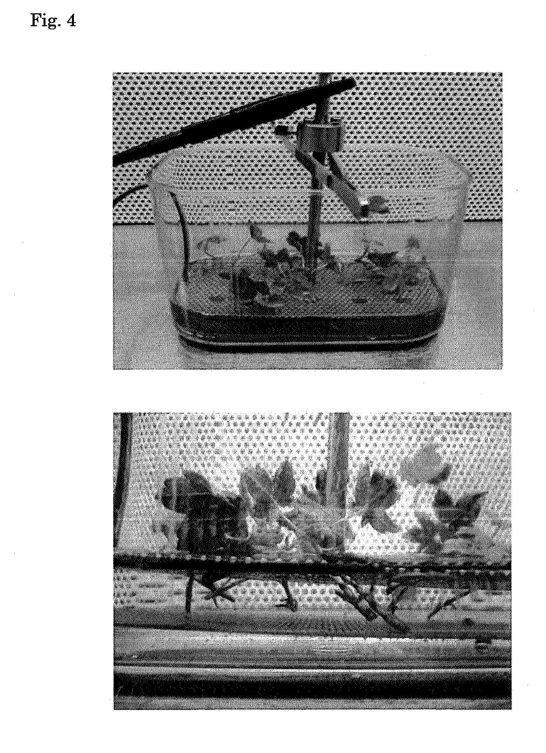

PRF treatments were performed in plastic containers with electrode plates, leaving 1.5 cm space between the upper and the lower electrodes.
The small container contains 150 ml of a liquid tissue culture medium and the bigger container 250 ml (see Fig. 1).
Alternatively, autoclavable electrodes which fitted into petridishes, were used for treating tissue culture plants with PRF under sterile conditions (Fig. 2 and 3). For PRF treatment the plants were put as whole plants into the space between the electrodes of the smaller container, or in a second method, they were put upright into holes in the bigger container, so that the shoots were sticking above, but the stems were exactly between the electrode plates (Fig. 4).
For each treatment at least 10 tissue culture derived plants were used.

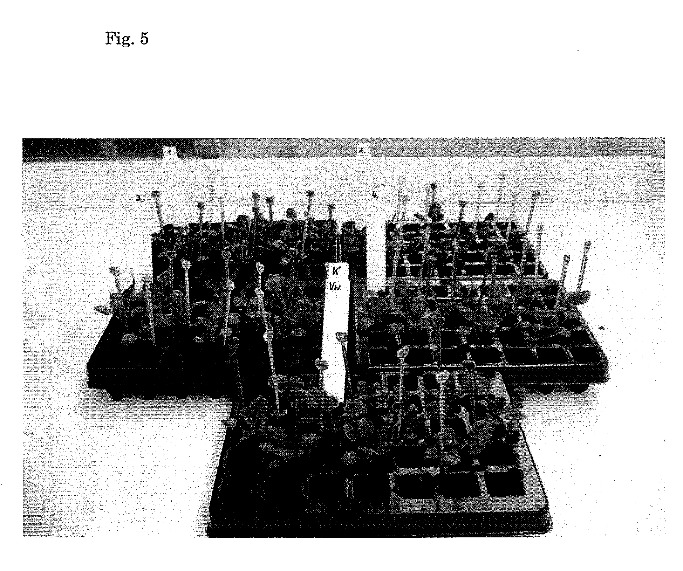

After the PRF treatment, the tissue culture plants were transferred to a tray with soil and covered with a plastic hood to avoid excessive water loss for the first 2 weeks. After this weaning period, the plants that had grown roots were transferred to the greenhouse or potted separately. Evaluations were done twice a week for a period of 6 weeks. Plants in different developmental stages were given a specific coloured stick (Fig. 5).

### Results

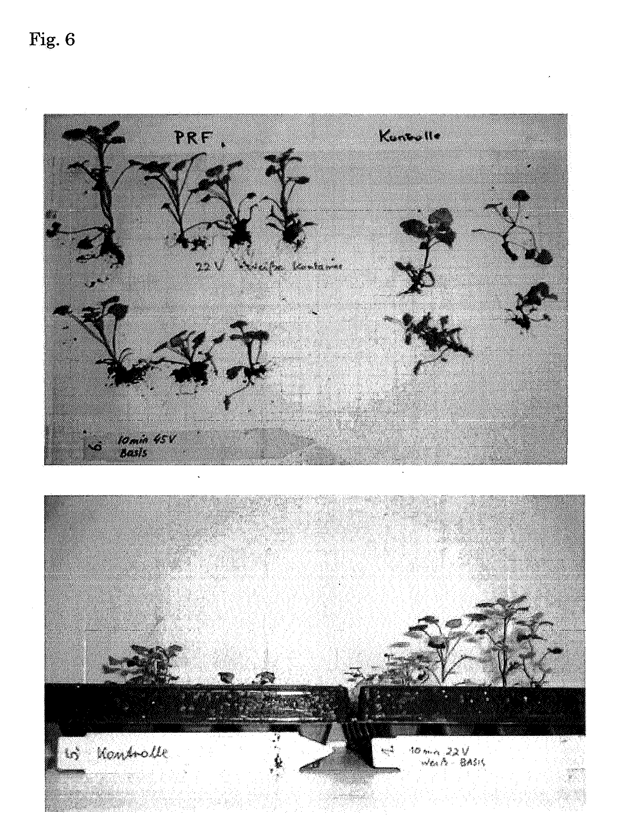

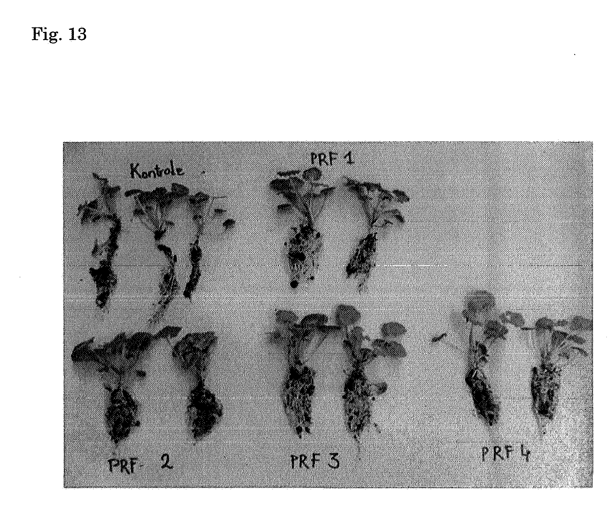

The plants that were treated with PRF were in general more homogenous (in size and in developmental stages), they were bigger and more vigorous than non treated plants (Fig. 5).
The PRF treated plants developed roots faster and the roots were more abundant (adventitious) and better developed than the untreated controls, so that they could survive easier in the soil (Fig. 6, Fig. 12, Fig. 13 ).
The plants which did not form roots at all, decayed after 4 weeks.

The optimum PRF treatment is different for each plant species. For the flowering plants, in general a treatment with 15V/cm for 10 minutes was the best, whereas at higher than 30V/cm the plant quality was decreased.
Also a second PRF treatment, 2 hours after the first treatment, with the lowest dosage (15V for 10 minutes) did not affect the quality of the plants, but it did not improve the results significantly.
Homogeneity of the flowering plants was found to be increased after PRF treatment, with an optimum at 15 V/cm for 10 minutes and it was decreased when treated with voltages higher than 23V/cm for 10 minutes.

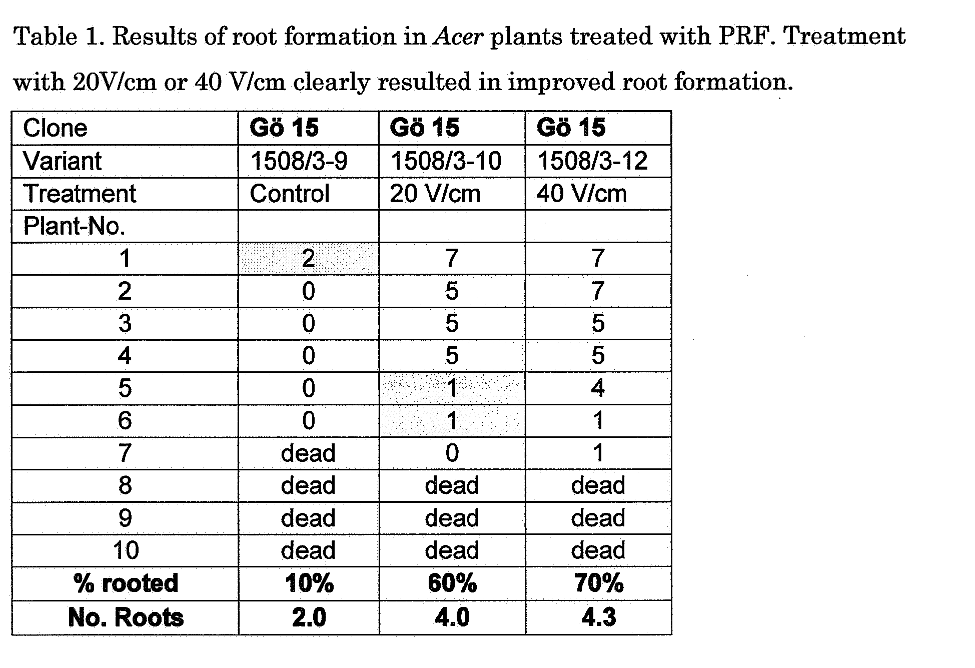

### Example 2 Description of the methods for plant cells to induce cell divisions, shoot and root formation, by PRF treatment

Plant cells, derived from a flower bulb, were cultured as cell suspensions in liquid MS medium with 2 mg/l NAA and 30 g/l sucrose. In this culture medium, the plant cells are completely dedifferentiated as single cells or a cluster of cells (showing no shoots or roots). Just before the PRF treatment, small cell clumps of about 20-50 cells were put on fresh agar medium (Murashige & Skoog medium with 0.6% agar, 3% sucrose).
Some of the plates contained phytohormones (1 mg/l BA and 0.2 mg/l IAA, which is necessary for shoot regeneration) and some of the plates were without any hormones at all.
The cell clumps were put into two rows on the agar, one row was treated (+) and the other was not treated (-) with PRF.

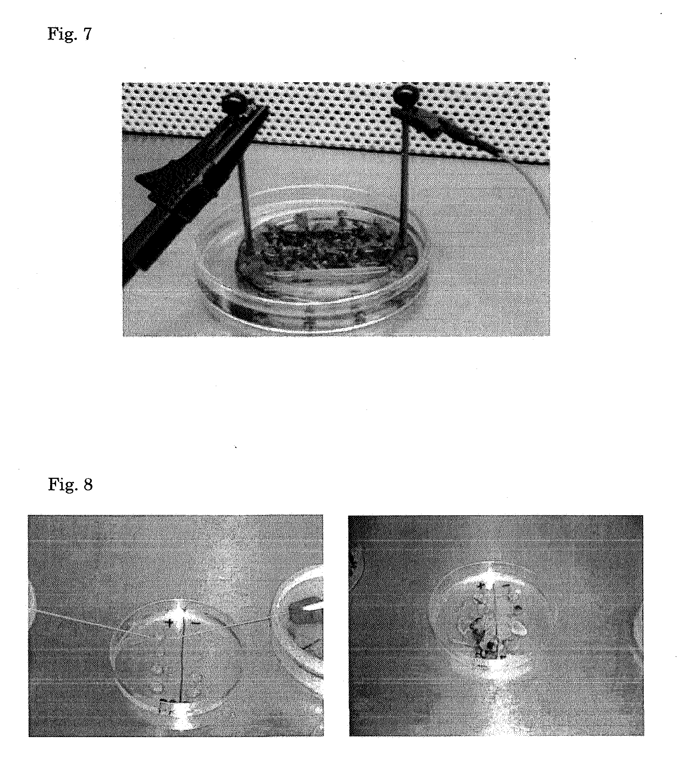

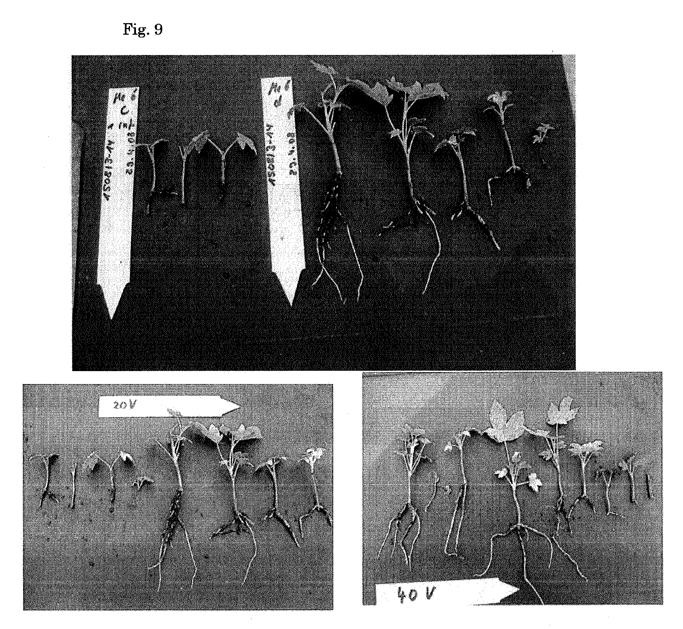

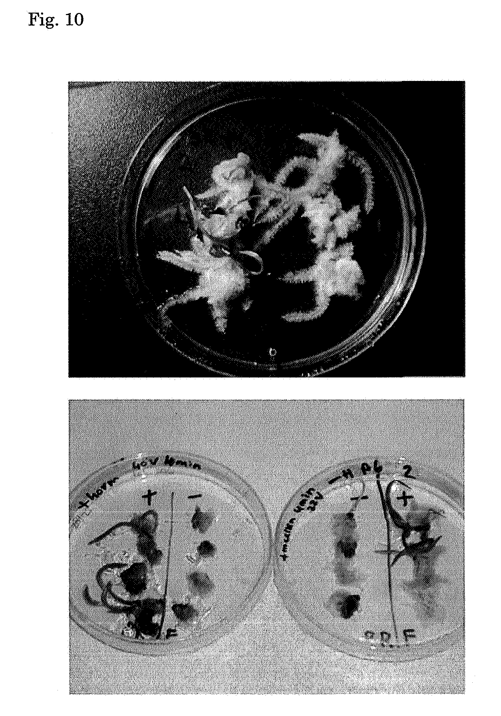

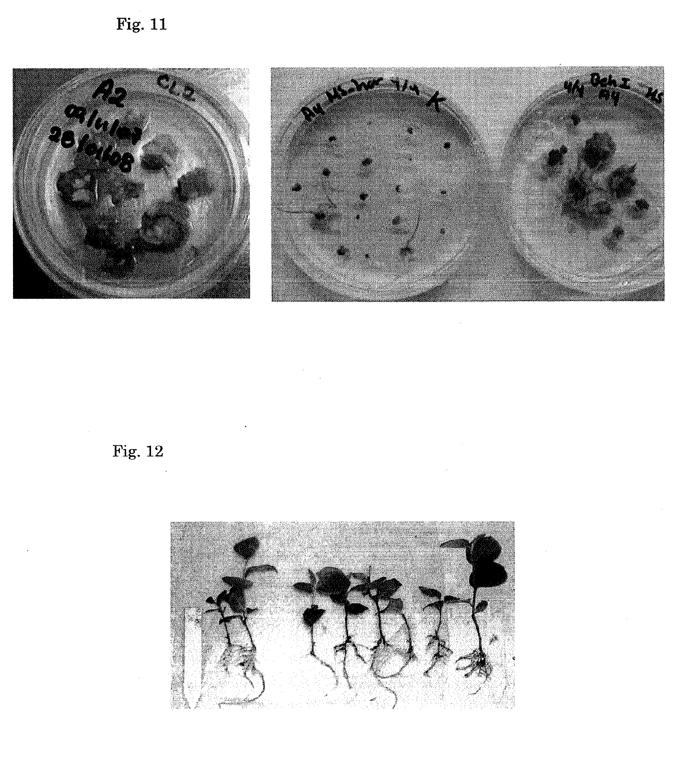

For treating the plant cells, we have used 2 sterile CXE-10 needles (Cotop International), with an active tip of 5 mm. The tip of the needles was injected into the cell clumps (Fig. 7), or were put just in front of these cells (when the cells were too sturdy to being injected, see Fig. 8, left panel).
These two needles were wired to the generator (Fig. 1). We have used pulses of 2 Hz, a pulse duration of 10 ms varying voltage settings (ranging from 15-30 V/cm) and varying duration of the PRF treatment (ranging from 2-5 minutes). Impedance of the solid agar medium was found to be 380 Ohm.
In another experiment we have put the cells between electrodes instead of needles (Fig. 9)
After the PRF treatment, the plant cells were kept cultured in sterile petridishes, which were incubated in a climate chamber (with temperature control, under normal light/dark conditions) on media without hormones. Observations were done weekly and plant growth as well as root and shoot formation were evaluated (Fig. 10, 11).

### Results

No significant differences were found between the treatments by which needles were pierced into the cells or by putting the needles just outside the cells or by using electrodes.
In all treatments, a faster growth and even shoot differentiation was observed after PRF treatment, compared to the untreated controls. This effect was independent of the media used (with or without hormones). This indicates, that plant hormones may not be necessary to induce shoot and root formation in PRF treated cells.

Depending on the tissue and the goal of the treatment, the voltage was varied between 25-50 V/cm and the duration of each treatment between 2-15 minutes. PRF can be applied in plant tissue culture as a one time treatment or in subsequent treatments over time. This needs to be optimized for each plant species. In our studies of the plant cells, the optimal treatment for plant cells was found to be between 15 and 30 V/cm during 2-4 minutes. PRF treated cells showed increased root formation (thicker and more adventitious root formation; Fig. 9, 10, 11) than untreated cells, even in medium without hormones (Table 1). No harmful effects were found in the range of tested Voltages and exposure time

## Claims

1. Method to increase growth or differentiation in vitro in a cell or tissue culture comprising culturing said cell(s) or tissue and applying PRF to said cell or tissue.

2. Method according to claim 1, wherein the cells or tissue are derived from a plant or an animal.

3. Method according to claim 1 or 2, wherein said cell is an undifferentiated cell.

4. Method according to any of the previous claims, wherein the cells or tissue are derived from a plant and in which differentiation implies the outgrowth of roots and/or shoots or of somatic embryos.

5. Method according to any of the previous claims, wherein the parameters for PRF are as follows:
a. Frequency: 50,000-1,000,000 Hz, preferably 150,000 - 500,000 Hz
b. Pulse duration: 0.1 - 100 msec, preferably 5-20 msec.
c. Pulse frequency: 1 - 20/sec, preferably 1 - 3/sec
d. Voltage: 1-100 V per cm distance between electrodes
e. Exposure time: 2 - 180 minutes

6. Use of PRF for increasing the growth or differentiation in in *vitro* cell or tissue culture.

7. Cell or tissue culture treated according to a method according to any of claims 1-5.

8. Cell or tissue culture according to claim 7 which is derived from a plant.

9. Plant grown from a cell or tissue culture according to claim 8.

10. Cell culture according to claim 7 wherein the cells are derived from an animal.

11. Cell culture according to claim 10, wherein the animal is a human.
